# EUROPEAN PATENT APPLICATION

(11) **EP 2 783 630 A1**
(43) Date of publication of application: **01.10.2014**
(21) Application number: 13001568.8
(22) Date of filing: 27.03.2013
(51) Int. Cl.: A61B 5/103, A61B 5/11, A63B 69/00, A43B 5/06, G01C 22/00

(54) **Human motion analysis method and device**

(71) Applicant: ETH Zurich, 8092 Zurich (CH)
(72) Inventor: Adelsberger, Rolf, CH - 8304 Wallisellen (CH)

(57) **Abstract**

A human motion analysis method is executed by a human motion analysis device. The device comprises a first sensor device (1) and optionally a second sensor device (1'), each sensor device comprising a pressure sensor configuration arranged (2) in or on an insole or in or on a shoe, an inertial measurement unit (IMU) (3) and a data processing unit (4). The method comprises the steps of
● collecting measurement data from the pressure sensor configuration (2) and from the inertial measurement unit (3);
● computing, in the sensor device (1, 1'), pre-processed measurement data from the measurement data;
● computing one or more characteristics that describe a motion or a stance of a subject that is wearing the sensor device (1, 1').

## Description

The invention relates to a human motion analysis method and device as described in the preamble of the corresponding independent claims.

US6836744 (B1) shows a portable system for analyzing human gait, with a foot sensor system with pressure sensors, accelerometers and rate sensors. The system calculates maximum pressure, mean pressure and a pressure line, and is able to communicate with a PDA or PC.

US2002040601 (A1) and US5955667 (A) show a motion analysis system based on an accelerometer only.

US6882955 (B1) shows monitoring activity of a user, with pace determination and analysis bases on an accelerometer signal.

US2003009308 (A1) shows an instrumented insole with pressure, gyro and accelerometer sensors. Velocity, force and power are computed.

WO2012055029 (A1) shows a sensory replacement system based on pressure sensing. Pressure data from an insole is sent to other devices and is output to other body parts. Acceleration signals can be used, and a centre of a foot is calculated and used.

US2011275956 (A1) shows intelligent orthotic insoles with processing and wireless transmission of pressure maps, for diverse applications such as controlling an avatar, providing feedback to diabetes patients or to balance impaired persons.

NL1034258 (C2) shows a patient's foot load measuring method using a small number of discrete pressure sensors.

US2007125166 (A1) shows a system with an insole pressure measurement. An acoustic signal is triggered wirelessly, if the pressure exceeds a predetermined limit.

WO2012128801 (A1) shows an insole with pressure sensors (two or four per foot), accelerometers and a wireless transmitter. Information from the sensors can be collected and information from both feet can be displayed. A warning signal can be generated if a force exceeds a predetermined limit

S. Morris and J. Paradiso, "Shoe-integrated sensor system for wireless gait analysis and real-time feedback," in Engineering in Medicine and Biology, 2002. 24th Annual Conference and the Annual Fall Meeting of the Biomedical Engineering Society EMBS/BMES Conference, 2002. Proceedings of the Second Joint, vol. 3, oct. 2002, pp. 2468 - 2469 vol.3, presents a mobile system for measuring basic force distributions (amongst other modalities) on feet. The device is used for abnormality detection in gait or for fitting prostheses. However, it requires a tethered connection to a computer or to a hip-attached device.

S. Pfaffen, P. Sommer, C. Stocker, R. Wattenhofer, and S. Welten, "Planipes: Mobile Foot Pressure Analysis," in 1st International Workshop on Mobile Systems Applications, and Services for Healthcare (mHealthSys), Seattle, Washington, USA, November 2011, discloses a mobile version of a pressure sensing device. The device combines 16 FSRs (force-sensitive resistors) into a userfitted insole. The locations where the pressure is measured is pre-defined by a physician. The device transmits pressure data to a smart phone via bluetooth in realtime. There is no possibility for storing data in the device. The sensor device acts as a gateway for FSR values while all algorithms run on the mobile device.

It is an object of the invention to create a human motion analysis method and device of the type mentioned initially, which improves on and overcomes disadvantages of the methods and devices mentioned above. In particular, an object of the invention is to provide a human motion analysis method and device that allows to estimate the activity of a subject with a small set of sensors. A further object of the invention is to provide an unobtrusive human motion analysis device that can run autonomously for extended periods of time and alert a user when a particular stance or motion pattern or activity occurs, or store information about such an occurrence.

These objects are achieved by a human motion analysis method and device according to the corresponding independent claims.

The human motion analysis method is executed by a human motion analysis device, the device comprising a first and optionally a second sensor device, each sensor device comprising a pressure sensor configuration arranged in or on an insole or in or on a shoe, an inertial measurement unit (IMU) and a data processing unit. The method comprises the steps of
- collecting (typically by means of the data processing unit) measurement data from the pressure sensor configuration and from the inertial measurement unit;
- computing, in the sensor device (typically by means of the data processing unit), pre-processed measurement data from the measurement data, which can reduce the amount of data, i.e. the number of bits needed to represent the data;
- computing (typically by means of the data processing unit or another unit) one or more characteristics that describe a motion or a stance of a subject that is wearing the sensor device.

In an variant of the human motion analysis method, it comprises the step of storing pre-processed measurement data in the sensor device and/or of transmitting pre-processed measurement data to an external device or to another sensor device.

Since the pre-processed measurement data is computed in the sensor device itself, it can be made to require less space for storage and/or transmission. This in turn makes it possible to either store a useful set of information in limited storage means provided in the sensor device, or to transmit the pre-processed measurement data, for example in real time, over a communication channel with limited bandwidth. It thus becomes possible to implement the sensor device with small dimensions, low power requirements and thus small batteries, and without wired connections for communication and power supply.

In an variant of the human motion analysis method, it comprises the steps of
- computing in at least one sensor device (typically by means of the data processing unit) representative pressure values and representative inertial sensor values from the measurement data or from the pre-processed measurement data, and
- computing (for example by means of a classification unit) a classification of the activity of a subject that is wearing the sensor device from the representative pressure values and the representative inertial sensor values.

The classification unit can implement a support vector machine for classification, or any other known clustering method. Such clustering methods can be trained, in a learning phase, by associating value vectors with classification labels. Then, in use, a value vector can be input to the clustering method, and a classification label is returned.

In an variant of the human motion analysis method, the representative pressure values can be a mean pressure or a median pressure on the pressure sensor configuration, or a weighted sum of pressure values obtained from the pressure sensor configuration. In other variants, the representative pressure values can be the result of a principal components analysis of the 2D pressure distribution over the pressure sensor configuration, for example, vectors or scalar values corresponding to the direction or length of principal components (axes of ellipsoid fitting the pressure distribution. A further representative value can be the relation between the size of principal components, i.e. an indication of how round or elongated the pressure distribution is.

In an variant of the human motion analysis method, the representative inertial sensor values are magnitudes of acceleration.

In variants of the human motion analysis method, the classification is based on one or more of
- a single scalar representative pressure value and a single representative inertial sensor value, or
- on a sequence over time of scalar representative pressure values and scalar representative inertial sensor values.

The single scalar representative pressure value and a single representative inertial sensor values can be obtained by filtering the measurement data or from the pre-processed measurement data, e.g. by combining measurement data from a window in time, such as, e.g., in a moving average filter.

In an variant of the human motion analysis method, the activity is classified as one of sitting, walking and standing.

For example, walking corresponds to a high mean pressure and a high accelerometer magnitude. Standing corresponds to a high mean pressure and a low accelerometer magnitude is low. Sitting corresponds to a medium or low mean pressure and low accelerometer magnitude. With regard to location, the mean pressure can be computed over the two feet or from a single foot. With regard to time, the mean can be computed as an average or a weighted average or a filtered value over a time window.

In an variant of the human motion analysis method, it comprises the step of evaluating the characteristics (that describe a motion or a stance of a subject that is wearing the sensor device) and, in the event that the characteristic satisfies a evaluation criterion transmitting (typically by means of a communications interface) a corresponding status signal to an external device, or storing a corresponding status information (in the sensor device, for example) for later retrieval and transmission to an external device.

Such an evaluation criterion (or predetermined condition) regarding pressure and IMU data from a single device, corresponding to a foot, can be, for example:
- the acceleration and / or the pressure remain under a limit for longer than a maximum time;
- the variance of the step frequency exceeds a given limit;
- the range of motion of the feet changes, while the step frequency or the step frequency variation changes or remains the same.
- the mean pressure increases significantly over multiple steps while the IMU data suggests a flat surface. This in an indication for unbalanced loading.
- the combined IMU and pressure data indicates or suggests an activity that should or should not be performed, e.g., a knee patient running or standing too long etc.

In an variant of the human motion analysis method, it comprises the steps of combining, by means of an aggregator, unit pre-processed measurement data from at least two sensor devices,
- the aggregator unit either being a further device, which can be in wireless or wired communication with the at least two sensor devices, receives the pre-processed measurement data from each of the at least two sensor devices,
- or the aggregator unit being part of one of the at least two sensor devices that receives the pre-processed measurement data from the other sensor device(s).

For this purpose, each sensor device can comprise a communication unit, the communication unit transmitting, wired or wireless, preprocessed measurement data to an aggregator unit. One of the sensor devices can operate as an aggregator unit, receiving pre-processed measurement data from at least the other sensor device and combining the received data with its own pre-processed measurement data.

In an variant of the human motion analysis method, it comprises the step of computing a centre of pressure of two sensor devices, the centre of pressure being at least an approximation of the average of the locations of the sensor devices, each location weighted by the sum of pressures measured at that location by the respective sensor device. Alternatively, or in addition, the location can be weighted with weighting coefficients derived from data from the IMU system. For example, the magnitude of the acceleration, or one or more components of the acceleration in particular spatial directions can be used for wighting.

In an variant of the human motion analysis method, it comprises the step of determining a trajectory in space (typically in two dimensions) and/or over time of the centre of pressure and optionally also computing characteristics of this trajectory.

Such characteristics can be, for example, spatial or temporal frequency, amplitude etc. of this trajectory. Further characteristics can be determined from a multi-time-window analysis. Characteristics can be parameters of a Brownian motion model describing the trajectory. Characteristics can be parameters from 2D shape analysis, 3D shape analysis, analysis of trajectories over time, etc.

In an variant of the human motion analysis method, it comprises the steps of evaluating the characteristics of the trajectory and, in the event that the characteristic satisfies an evaluation criterion (or a predetermined condition, typically by means of a communications interface) transmitting a corresponding status signal to an external device, or storing (for example, in the sensor device itself) a corresponding status information for later retrieval and transmission to an external device.

Such an evaluation criterion (or predetermined condition) regarding pressure and IMU data from two devices, corresponding to two feet, can be, for example:
- stability. Stability can be determined by estimating parameters of a Brownian motion model fitted to the sensor data. Such data can comprise a trajectory of pressure over time or of IMU data such as spatial location or acceleration over time, or of other combinations of sensor data. Brownian motion parameters are correlated with stability and are indicative of stability.
- Activity detection. For example, activities detected can comprise one or more of: sitting, standing, walking, variations of walking such as running, climbing or descending stairs, climbing or descending ladders, biking, or other sports
- Activity rating: performance assessment of the aforementioned activities: for example, how are forces and/or power distributed over two feet while biking/running/walking/...
- Exhaustion detection.
- Fall detection, accident detection.
- Rehabilitation tracking: monitoring exercises assigned by a therapist.

In an variant of the human motion analysis method, it comprises the steps of detecting whether a particular motion pattern occurs, and when this is the case,
- either communicating to an external device that this motion pattern has occurred, optionally communicating further data that characterises this motion pattern,
- and/or storing corresponding information (e.g. in the sensor device with the aggregator) for later retrieval and transmission to the external device,
- and/or enabling a corresponding indicator signal
- and/or displaying a corresponding message on an output device.

Such motion patterns can be detected on the basis of variance in step frequency, changes in load patterns. Such load patterns can be assessed within one foot (intra-foot load pattern) or over two feet (inter-feet or between-feet load pattern). Further characteristics of motion patterns that can be observed are "heel time", "toe time", "stance time" (i.e. the absolute or relative times that heels or toes etc. carry the main pressure), etc.

The signal communicated to the external device can be transmitted by means of a wireless near field communication protocol such as WLAN, Bluetooth, ANT+ or the like, or by means of a SMS (short message system) text message or an e-mail.

The indicator signal can be a visual indicator such as a light, or an acoustic signal or a vibration signal or a combination of one or more of these. The output device can be a graphic display such as an LED screen or electronic paper, or a (micro)mechanically driven indicator.

The human motion analysis device comprises a first sensor device and optionally a second sensor device, each sensor device comprising a pressure sensor configuration arranged in or on an insole or in or on a shoe, an inertial measurement unit (IMU) and a data processing unit. The human motion analysis device is configured and programmed to perform the method as described above.

Further embodiments are evident from the dependent patent claims. Features of the method claims may be combined with features of the device claims and vice versa. The subject matter of the invention will be explained in more detail in the following text with reference to exemplary embodiments which are illustrated in the attached drawings, in which:
- Figure 1: shows a single sensor device;
- Figure 2: schematically shows the structure of a single sensor device;
- Figure 3: schematically shows two sensor devices interacting with an external device;
- Figure 4: schematically shows two sensor devices interacting with each other; and
- Figure 5: a centre of pressure and its trajectory projected into 2D-space.

The reference symbols used in the drawings, and their meanings, are listed in summary form in the list of reference symbols. In principle, identical parts are provided with the same reference symbols in the figures.

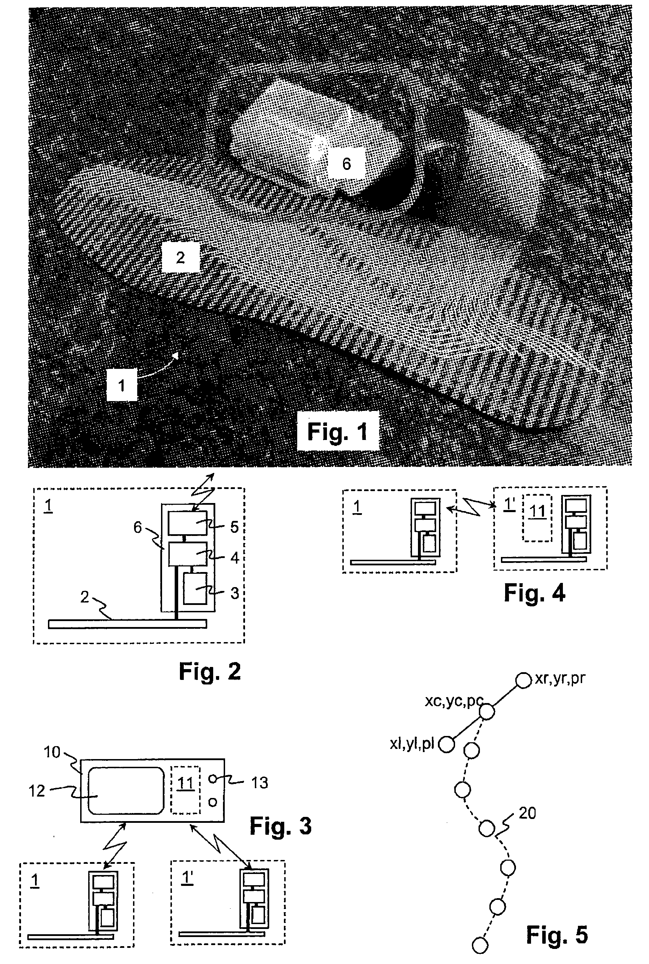

**Figure 1** shows a single sensor device 1. The sensor device 1 comprises a pressure sensor 2 in the shape of an insole. It can be integrated in an insole, or be made part of a shoe. The sensor device 1 further comprises a board unit or simply board 6 with further sensors, data processing and communication means. **Figure 2** schematically shows the structure of a single sensor device, with the board 6 comprising an inertial measurement unit 3, a data processing unit 4 and a communication unit 5. The inertial measurement unit 3 typically comprises a three axis accelerometer and a three axis gyro, and optionally magnetometers. The data processing unit 4 is a microprocessor and comprises a memory for storing digital data. The communication unit 5 allows for wireless communication, e.g. according to the ANT+ standard.

**Figure 3** schematically shows two sensor devices 1, 1' interacting with an external device 10 through a wireless communication link. The external device 10 can be a smartphone or PDA or personal computer or wearable computer or a data processing and storing unit dedicated to be interoperable with the sensor devices 1, 1'. The external device 10 comprises an aggregator 11 for storing, processing and combining data received from the sensor devices 1, 1', and may also comprise a display 12 and/or a visual indicator 13, such as an LED or a mechanical indicator.

**Figure 4** schematically shows two sensor devices 1, 1' interacting with each other through a wireless communication link. The aggregator 11, with essentially the same functions as described above, is implemented in a second one of the two sensor devices 1, 1', and receives data from the first sensor device 1 over the communication link, and from the sensors of the second sensor device 1' directly.

**Figure 5** shows a centre of pressure and its trajectory. Coordinates of the location of the centre of pressure in two dimensions, e.g. on the ground on which a subject is walking, are labelled as xc, yc, and the pressure at the centre of pressure is labelled as pc. The location of the centre of pressure is computed from the location and pressure of a left foot (xl, yl, pl) and a right foot (xr,yr,pr). This can be done by computing a weighted average of the location coordinates, the weight being proportional to the respective pressure. Recording the centre of pressure over time results in a trajectory 20 of the centre of pressure over time and through (2D) space.

The pressure pl, pr from each foot can be computed as the sum of pressure values from the corresponding pressure sensor 2. The location of each foot can be determined from measurements made by the inertial measurement unit 3. The inertial measurement units 3 of the two feet can be calibrated, and their relative position determined, in a calibration phase in which the subject wearing the sensor devices 1, 1' takes on a specific pose, such as standing upright.

Exemplary steps in processing the sensor data from a single sensor device 1, starting with measurement data obtained from its sensors, are:
- collecting **measurement data** from the pressure sensor configuration and from the inertial measurement unit. Such data can be the pressure distribution over the pressure sensor, i.e. over the subject's foot.
- computing **pre-processed measurement data.** Such data can be the a reduced set of pressure values (e.g. reduced resolution over the sensor area, e.g. one pressure value for the front foot and one for the back foot), or mean pressure and center of pressure and/or principal axis of pressure distribution. Furthermore, such data can also comprise time-domain filtered data, and/or characteristic parameters or coefficients from a data compression or transform method (e.g. Fourier or wavelet coefficients). With regard to IMU data, such pre-processed data can be filtered or down sampled acceleration or location data, optionally calibrated with reference data from a calibration step, etc.
- computing **representative pressure values and representative inertial sensor values.** Such data can be a single total or mean pressure value for the entire foot, and the magnitude of an acceleration vector, computed from the 3D acceleration determined from the IMU.
- computing (for example by means of a classification unit) a **classification** of the activity of a subject that is wearing the sensor device. This classification can be, for example, one of "walking", "standing", "sitting", "climbing stairs", etc. This classification is a characteristic describing a motion or a stance of the subject.

An optional further step is:
- evaluating the characteristics and, in the event that the characteristic satisfies a evaluation criterion, transmitting a corresponding status signal or storing a record of this event. For example, if pressure and acceleration remain below a certain threshold for a certain time, this indicates that the subject has not moved and may need assistance.

The step of computing pre-processed measurement data can involve the following processing steps: filtering (spatial AND temporal), binning, convex hull estimation of patches, principal component analysis (PCA).

The classification as described above, can be based on the following procedure: The classification algorithm, in an embodiment, discriminates between standing, walking and sitting episodes, using the accelerometer magnitude and mean pressure as features. In a first step the system calculates the accelerometer magnitude. From the pressure sole sensor 2 the mean pressure value is calculated for every sample time. To be less susceptible from noise the feature on the accelerometer's magnitude and the pressure-mean feature are calculated within a 500ms window prior to being fed to the classifier. The class boundaries are intuitively clear: walking is high mean pressure and high accelerometer magnitude. Standing differs from walking as accelerometer magnitude is low. Sitting shows medium or low pressure mean with low accelerometer magnitude. Due to the characteristics of the feature space, the classifier should be of higher order, e.g. k-means, SVM, HMM etc.. The support vectors (for SVM) or other model-dependent parameters (e.g. for HMM etc.) can be pre-calculated or can be learned at run-time. Further, higher-level activities like "standing up" or "sitting down" can be estimated from base activities like "sitting" → "standing"/"walking" and transitions between such base activities.

Exemplary steps in processing the sensor data from two sensor devices 1, 1', one on each foot of the subject, starting with measurement data obtained from its sensors, are:
- in each sensor device 1, 1' collecting measurement data as described above.
- in each sensor device 1, 1' computing pre-processed measurement data as described above.
- optionally, in each sensor device 1, 1', computing representative pressure values and representative inertial sensor values as described above.
- transmitting the pre-processed measurement data or the representative values to the aggregator, the aggregator residing in a further ("external") device or in one of the sensor devices 1, 1'.
- combining the information received by the aggregator, and computing a characteristic that describes a motion or a stance of the subject based on information from both feet. This can be done by computing the centre of pressure of the subject, determining the centre's trajectory in space and/or time and computing one or more characteristics of this trajectory.

An optional further step is, similar to the above,
e valuating the characteristics and, in the event that the characteristic satisfies a evaluation criterion, transmitting a corresponding status signal or storing a record of this event.

While the invention has been described in present embodiments, it is distinctly understood that the invention is not limited thereto, but may be otherwise variously embodied and practised within the scope of the clailns.

## Claims

1. A human motion analysis method executed by a human motion analysis device, the device comprising a first sensor device (1) and optionally a second sensor device (1'), each sensor device comprising a pressure sensor configuration (2) arranged in or on an insole or in or on a shoe, an inertial measurement unit (IMU) (3) and a data processing unit (4), the method comprising the steps of
• collecting measurement data from the pressure sensor configuration (2) and from the inertial measurement unit (3);
• computing, in the sensor device (1, 1'), pre-processed measurement data from the measurement data;
• computing one or more characteristics that describe a motion or a stance of a subject that is wearing the sensor device (1, 1').

2. The human motion analysis method of claim 1, comprising the steps of
• computing in at least one sensor device representative pressure values and representative inertial sensor values from the measurement data or from the pre-processed measurement data, and
• computing, a classification of the activity of a subject that is wearing the sensor device (1, 1') from the representative pressure values and the representative inertial sensor values.

3. The human motion analysis method of claim 2, wherein the representative pressure values are a mean pressure or a median pressure on the pressure sensor configuration, or a weighted sum of pressure values obtained from the pressure sensor configuration.

4. The human motion analysis method of claim 2, wherein the representative inertial sensor values are magnitudes of acceleration.

5. The human motion analysis method of one of claims 2 to 4, wherein the classification is based on a single scalar representative pressure value and a single representative inertial sensor value or on a sequence over time of scalar representative pressure values and scalar representative inertial sensor values.

6. The human motion analysis method of one of claims 2 to 5, wherein the activity is classified as one of sitting, walking and standing.

7. The human motion analysis method of one of claims 1 to 6, comprising the step of evaluating the characteristics and, in the event that the characteristic satisfies an evaluation criterion, transmitting a corresponding status signal to an external device (11), or storing a corresponding status information for later retrieval and transmission to an external device (11).

8. The human motion analysis method of claim 1, comprising the step of combining, by means of an aggregator unit (11) pre-processed measurement data from at least two sensor devices (1, 1'),
• the aggregator unit (11) either being in a further device (10) that receives the pre-processed measurement data from each of the at least two sensor devices (1, 1'),
• or the aggregator unit (11) being part of one of the at least two sensor devices (1; 1') that receives the pre-processed measurement data from the other sensor device(s) (1'; 1).

9. The human motion analysis method of claim 8, comprising the step of computing a centre of pressure (xc, yc) of two sensor devices (1, 1'), the centre of pressure being at least an approximation of the average of the locations (xl, yl, xr, yr) of the sensor devices, each location weighted by the sum of pressures measured at that location (pl, pr) by the respective sensor device (1, 1').

10. The human motion analysis method of claim 9, comprising the step of determining a trajectory in space and/or over time of the centre of pressure and computing characteristics of this trajectory.

11. The human motion analysis method of one of claims 8 to 10, comprising the step of evaluating the characteristics and, in the event that the characteristic satisfies an evaluation criterion, transmitting a corresponding status signal to an external device, or storing a corresponding status information for later retrieval and transmission to an external device.

12. The human motion analysis method of one of claims 8 to 11, comprising the step of detecting whether a particular motion pattern occurs, and when this is the case,
• either communicating to an external device that this motion pattern has occurred, optionally communicating further data that characterises this motion pattern,
• and/or storing corresponding information for later retrieval and transmission to the external device,
• and/or enabling a corresponding indicator signal
• and/or displaying a corresponding message on an output device.

13. A human motion analysis device comprising a first sensor device (1) and optionally a second sensor device (1'), each sensor device comprising a pressure sensor configuration arranged in or on an insole or in or on a shoe, an inertial measurement unit (IMU) and a data processing unit, the human motion analysis device being configured and programmed to perform the method of one of the preceding claims.
